(19) Europäisches Patentamt
European Patent Office
Office européen des brevets

(11) Numéro de publication: **0 024 241**
**B1**

# (12) FASCICULE DE BREVET EUROPEEN

(45) Date de publication du fascicule du brevet: **14.03.84**

(21) Numéro de dépôt: **80401167.4**

(22) Date de dépôt: **08.08.80**

(51) Int. Cl.³: **C 07 D 307/32**, C 07 D 307/93 // C07D307/58

(54) **Nouveaux composés sulfonés comportant un cycle lactonique, ainsi qu'un procédé de préparation de dérivés cyclopropaniques.**

(30) Priorité: **10.08.79 FR 7920477**

(43) Date de publication de la demande: **25.02.81 Bulletin 81/8**

(45) Mention de la délivrance du brevet: **14.03.84 Bulletin 84/11**

(84) Etats contractants désignés: **AT BE CH DE GB IT LI NL SE**

(56) Documents cités:
**JOURNAL OF ORGANIC CHEMISTRY, vol. 39, no. 1, 11 janvier 1974, Washington US K. OHGA et al.: "Photoinduced addition of isopropyl alcohol to alpha, *beta-unsaturated lactones" pages 106-108***

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Martel, Jacques, 15, rue Douvillez, F-94140 Bondy (FR)**
Inventeur: **Tessier, Jean, 30, rue Jean Moulin, F-94300 Vincennes (FR)**
Inventeur: **Demoute, Jean-Pierre, 249bis, rue de Rosny, F-93100 Montreuil sous Bois (FR)**

(74) Mandataire: **Burlot, Pierre et al, Boite postale no 9 102, route de Noisy, F-93230 Romainville (FR)**

**EP 0 024 241 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Nouveaux composés sulfonés comportant un cycle lactonique, ainsi qu'un procédé de préparation de dérivés cyclopropaniques

La présente invention concerne de nouveaux composés sulfonés comportant un cycle lactonique, ainsi qu'un procédé de préparation de dérivés cyclopropaniques.

L'invention a plus précisément pour objet sous forme trans en positions 4 et 5, de configuration (4SP, 5RS), (4S, 5R) ou (4R, 5S), les composés de formule générale:

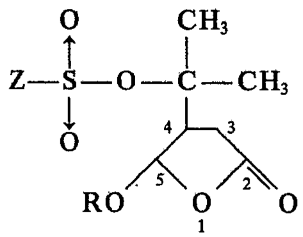

$(I_A)$

dans laquelle Z représente un radical alcoyle comportant de 1 à 4 atomes de carbone, un radical aryle monocyclique éventuellement substitué soit par un groupement alcoyle comportant de 1 à 4 atomes de carbone, soit par un groupement alcoxyle comportant de 1 à 4 atomes de carbone, soit par un atome d'halogène, soit par un groupement nitro et R représente le reste organique du menthol ou de l'alcool m-phénoxybenzylique.

Dans les composés de formule (I), Z représente notamment un méthyle, un éthyle, un propyle, linéaire ou ramifié, un butyle linéaire ou ramifié, un phényle, un phényle substitué soit par un méthyle, un éthyle, un propyle, linéaire ou ramifié ou un butyle linéaire ou ramifié, soit par un méthoxy, un éthoxy, un propyloxy linéaire ou ramifié ou un butyloxy linéaire ou ramifié, soit par un atome d'iode, de brome, de chlore ou de fluor, soit par un groupement nitro.

L'invention a également pour objet un procédé de préparation des dérivés cyclopropaniques de configuration (1RS, 4RS, 5SR), (1S, 4S, 5R) ou (1R, 4R, 5S) de formule générale (V):

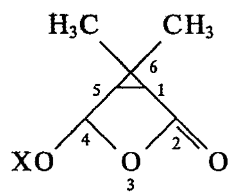

(V)

dans laquelle X représente un atome d'hydrogène ou le reste R d'un alcool R—OH éventuellement chiral, caractérisé en ce que l'on fait réagir, un composé de formule générale (II):

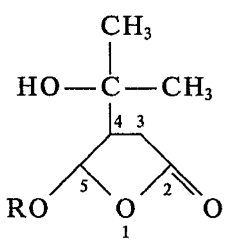

(II)

dans laquelle R représente le reste organique d'un alcool R—OH éventuellement chiral, le composé (II) étant de structure trans en position 4 et 5, et ayant dans le cas où R est le reste d'un alcool achiral une configuration (4 SR, 5 RS) et dans le cas où R est le reste d'un alcool chiral une configuration (4 S, 5 R) ou (4 R, 5 S), avec un composé de formule (III)

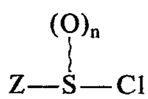

(III)

dans laquelle n = 1 ou n = 2, le trait ondulé représente une ou deux liaisons semi-polaires liant le ou les atomes d'oxygène à l'atome de soufre, suivant que n = 1 ou n = 2, et Z représente un radical alcoyle comportant de 1 à 4 atomes de carbone, un radical aryle monocyclique éventuellement substitué soit par un groupement alcoyle comportant de 1 à 4 atomes de carbone, soit par un groupement alcoxyle comportant de 1 à 4 atomes de carbone, soit par un atome d'halogène, soit par un groupement nitro,

— soit pour obtenir lorsque n = 1, un composé de structure trans en positions 4 et 5, de formule générale (IV):

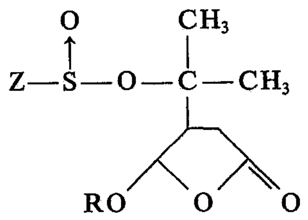

(IV)

dans laquelle R et Z conservent les significations précitées, que l'on fait réagir avec un agent oxydant, pour obtenir un composé de formule générale (I):

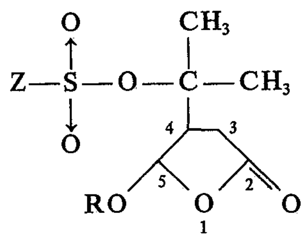

(I)

de structure trans en positions 4 et 5, de configuration (4SR, 5RS) si le reste d'alcool R est achiral ou de configuration (4S, 5R) ou (4R, 5S) si le reste d'alcool R est chiral,

— soit pour obtenir lorsque n = 2, un composé de formule générale (I) dans laquelle R et Z conservent les significations précitées, le composé de formule générale (I) étant de configuration (4SR, 5RS) si le reste d'alcool R est achiral et de configuration (4S, 5R) ou (4R, 5S) si le reste R est chiral, puis soumet à l'action d'un agent basique, un composé de formule générale (I), la cyclisation résultante ayant lieu avec rétention de configuration en ce qui concerne les corbones en positions 4 et 5 et faisant correspondre un composé V de configuration (1RS, 4RS, 5SR) à un composé I de configuration (4SR, 5RS) un composé V de configuration (1R, 4R, 5S) à un composé I de configuration (4S, 5R) et un composé V de configuration (1S, 4S, 5R) à un composé I de configuration (4R, 5S), puis hydrolyse, si désiré, en milieu acide, avec rétention de configuration, la fonction éther du composé V dans lequel X est un reste d'alcool.

L'utilisation d'un chlorure de sulfinyle (n = 1) conduit intermédiairement à un sulfinate IV.

Pour obtenir ce sulfinate, on opère avantageusement, en présence d'une base tertiaire et au sein d'un solvant choisi dans le groupe constitué par les hydrocarbures halogénés et les hydrocarbures aromatiques.

Dans un mode d'exécution préféré du procédé de l'invention, la base tertiaire est la pyridine et le solvant est le chlorure de méthylène.

Comme autre base tertiaire, on peut notamment utiliser la triéthylamine, la tripropylamine, la triphénylamine, la 2-méthyl 4-éthyl pyridine, la 3-éthyl 4-méthyl pyridine, la 2,4,6-triméthyl pyridine.

L'agent oxydant utilisé pour transformer les composés IV en composés I, est notamment le permanganate de potassium, l'eau oxygénée, l'acide peracétique ou l'acide paranitroperbenzoïque.

L'action de l'agent oxydant est effectuée de préférence au sein d'un solvant ou d'un mélange de solvants choises dans le groupe constitué par les hydrocarbures halogénés, les hydrocarbures aromatiques, l'acide acétique, l'acétone, les alcanols et l'eau.

L'utilisation d'un chlorure de sulfonyle (n = 2) conduit à l'obtention d'un sulfonate (I).

Pour obtenir ce sulfonate on opère avantageusement en présence de triéthylamine et au sein d'un solvant choisi dans le groupe constitué par les hydrocarbures halogénés et les hydrocarbures aromatiques.

Dans un mode d'éxécution préféré du procédé de l'invention conduisant au sulfonate, on utilise comme solvant le benzène.

L'agent basique utilisé pour effectuer la cyclisation est, de préférence, un hydroxyde alcalin.

Dans le procédé de l'invention, on fait agir de préférence, l'agent basique selon la méthode dite de catalyse par transfert de phase.

Dans un mode d'exécution préféré, dans la méthode dite de catalyse par transfert de phase, selon l'invention, le solvant est le chlorure de méthylène, le catalyseur est le chlorure de triéthylbenzyl ammonium, l'agent basique est la soude et l'on opère en présence d'eau.

L'hydrolyse du composé V dans lequel X représente le reste d'un alcool R – OH est effectuée par des méthodes connues en elles-mêmes.

Les composés II utilisés au départ de l'invention, lorsque R représente le reste d'un alcool achiral, sont préparés d'une manière générale, en faisant réagir un composé de formule (VI):

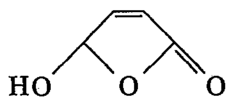

(VI)

avec un alcool achiral R—OH puis en soumettant le composé de formule (VII):

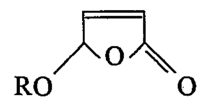

(VII)

résultant à l'action de l'isopropanol, en présence d'un promoteur radicalaire.

Un exemple de cette préparation est donné plus loin dans la partie expérimentale.

Lorsque les composés II sont optiquement actifs par leurs atomes de carbone chiraux en position 4 et 5, ils peuvent être préparés en faisant réagir sur le composé racémique (VI):

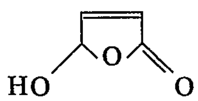

(RS)

(VI)

un alcool chiral $R_1OH$ pour obtenir un composé (VIII):

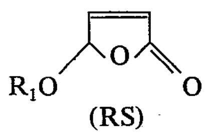

(RS)

(VIII)

en séparant ensuite les deux diastéréoisomères par une méthode physique, pour obtenir des composés $X_A$ et $X_B$:

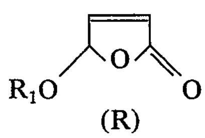

(R)

$X_A$

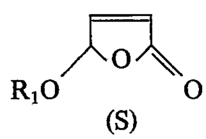

(S)

$X_B$

puis en faisant réagir sur le composé $X_A$ ou $X_B$ l'isopropanol, en présence d'un promoteur radicalaire, pour obtenir le composé II désiré, de configuration (4S, 5R) ou (4R, 5S).

Un exemple d'une telle préparation des composés II est donné plus loin dans la partie expérimentale.

Dans la méthode d'obtention des composés X dédoubles l'utilisation du 1-menthol ou (1R, 2S, 5R) 2-isopropyl-5-méthyl cyclobutyl-1-ol est avantageuse. La réaction d'éthérification entre le 1-menthol et la (5RS) 5-hydroxy 2,5-dihydrofuran-2-one, en milieu acide, conduit par induction asymétrique à un mélange de composés X plus riche en forme R qu'en forme S.

Une partie de la (5R) (—)-5-[(1R, 2S, 5R) 2-prop-2-yl-5-méthyl cyclohexyloxy] (5H) furan-2-one est insolubilisée dans le benzène. Un nouveau chauffage des liqueurs mères en milieu acide permet d'atteindre à nouveau l'équilibre entre les formes (R) et (S) et d'isoler, par insolubilisation, une nouvelle quantité d'isomère (R).

Au bout de trois ou quatre opérations successives, on obtient un rendement élevé en isomère R du composé X.

Le procédé de l'invention permet donc en deux ou trois étapes réactionnelles, à partir des composés II, eux-mêmes facilement accessibles de réaliser une synthèse stéréospécifique économique des composés de formule V et notamment du (1R, 4R, 5S) (—) 6,6-diméthyl 4-hydroxy 3-oxabicyclo [3.1.0] hexan-2-one ou du (1S, 4S, 5R) (+) 6,6-diméthyl 4-hydroxy 3-oxabicyclo [3,1,0] hexan-2-one.

Le procédé de l'invention présente, sur le plan chimique, un caractère inattendu. En effet, la préparation de sulfonates d'alcools tertiaires, difficile à réaliser pour des alcools fragiles comme les composés (II) a pu être effectuée avec des rendements satisfaisants grâce au type de réactions mises en jeu dans le procédé de l'invention. L'étape ultime de la synthèse, la cyclisation en composé V risquait de donner lieu à des réactions d'élimination avec formation de double liaison ou d'ouverture du cycle lactonique. Grâce aux conditions douces que permet le procédé de l'invention, les réactions secondaires ont pu être évitées et la cyclisation finale a été effectuée avec un bon rendement.

Les différents composés de formule V et tout particulièrement ceux pour lesquels X représente un

atome d'hydrogène, connus par le brevet français 1 580 474, permettent la préparation de plusieurs acides cyclopropane carboxyliques diversement substitués, acides qui, par estérification, conduisent à des produits insecticides très actifs.

Les exemples suivants illustrent l'invention sans la limiter.

Exemple 1

4-(2-méthyl sulfonyloxy prop-2-yl)5-[(3-phénoxy phényl)méthoxy]tétrahydro-furan-2-one (trans dl)

Dans 17 cm³ de benzène, on introduit 1,712 g de 4-(2-hydroxy prop-2-yl) 5-(3-phénoxy phényl)méthoxy tétrahydro-furan-2-one (trans dl) refroidit le mélange réactionnel à +5°C, ajoute 1,4 cm³ de triéthylamine, introduit lentement à +5°C, 0,78 cm³ de clorure de méthane sulfonyle en solution dans 6 cm³ de benzène, agite pendant 15 minutes à +5°C, ajoute une solution aqueuse d'acide chlorhydrique N, décante, lave à l'eau la phase organique, la sèche, concentre à sec et obtient 3 g de 4-(2-méthyl sulfonyloxy prop-2-yl)5-[(3-phénoxy phényl)méthoxy]tétrahydrofuran-2-one (trans dl) brut utilisable tel quel.

Spectre IR (Chloroforme)

Absorption à 1786 $cm^{-1}$ caractéristique du carbonyle de la γ lactone,

Absorption à 1338 $cm^{-1}$, 1172 $cm^{-1}$, carctéristique de $-SO_2$

Spectre de RMN (deutérochloroforme)

Pics à 1,57 — 1,60 ppm attribués aux hydrogènes des méthyles portés par le carbone en α de l'oxygène du groupement sulfonyle,

Pic à 2,58 ppm attribué aux hydrogènes en positions 3 et 4 de la furanone,

pic à 2,86 ppm attribué aux hydrogènes du méthyle du groupement méthanesulfonyle,

pics à 4,45 — 4,63 et 4,73 — 4,92 ppm attribués aux hydrogènes du méthylène en α du phénoxy phényle quadruplet à 4,68 ppm (J = 11 Hz),

pic à 5,5 ppm attribué à l'hydrogène en position 4 de la furanone,

pics à 6,83 — 7,5 ppm attribués aux hydrogènes des noyaux aromatiques.

La 4-(2-hydroxy prop-2-yl)5-(3-phénoxyphényl)méthoxy tétrahydrofuran-2-one(trans dl) peut être préparée de la manière qui suit:

Stade A

5-(3-phénoxyphényl)methoxy 2-(5H)furanone

On mélange 12 g de 5-hydroxy 2-(5H)furanone, 250 cm³ de benzène, 25 g d'alcool métaphénoxy benzylique et 200 mg d'acide paratoluène sulfonique, agite en distillant du benzène et le remplaçant plusieurs fois par la même quantité de benzène sec dans le mélange réactionnel. Après deux heures, on laisse refroidir à température ambiante et lave avec une solution saturée de bicarbonate de sodium puis à l'eau, sèche, concentre à sec sous pression réduite, obtient 39,7 g d'une huile que l'on purifie par chromatographie sur silice en éluant par un mélange benzène-acétate d'éthyle 9 — 1 et recueille 24,9 g du produit attendu.

Spectre de RMN

Pics à 7,3 — 7,4 ppm attribués aux hydrogènes en 4 de la furanone,

Pics à 6,18 — 6,32 ppm attribués aux hydrogènes en 3 de la furanone,

Pics à 6,03 ppm attribué à l'hydrogène en 5 de la furanone,

Pics à 4,58 — 4,76 et 4,85 — 5,12 ppm attribués aux hydrogènes du méthoxy,

Pics à 6,92 à 7,5 ppm attribués aux noyaux aromatiques.

Stade B

4-(2-hydroxy prop-2-yl) 5-(3-phénoxy phényl) méthoxy) tétrahydro furan-2-one (trans dl)

On chauffe au reflux sous agitation et sous atmosphère inerte, 3,5 g de 5-(3-phénoxy phényl) méthoxy 2,5-dihydrofuran-2-one dans 100 cm³ d'isopropanol, ajoute régulièrement en une heure et demie, par portions de 25 mg, 300 mg de peroxyde de benzoyle, concentre à sec sous pression réduite à 40 — 50°C et chromatographie le résidu dur silice en éluant avec un mélange benzène-acétate

d'éthyle (8–2). On recueille 3,7 g de produit brut qui repris par l'éther isopropylique donne des cristaux blancs. F = 50 – 55° C.

Analyse: $C_{20}H_{22}O_5$: 342,59
Calculé: C 70,16 H 6,4%
Trouvé: C 69,9 H 6,5%

Spectre de RMN
Pics à 1,18 et 1,21 attribués aux hydrogènes des méthyles,
pics à 2,16 à 2,75 attribués aux hydrogènes en 3 et 4 du cyclopentyle,
pics à 4,48 – 4,35 et 4,8 – 5 attribués aux hydrogènes du méthylène benzylique,
pics à 5,53 – 5,58 attribués aux hydrogènes en 5 du cyclopentyle,
pics à 6,83 à 7,5 attribués aux hydrogènes des noyaux aromatiques,
Pic à 1,5 attribué à l'hydrogène de l'hydroxyle.

## Exemple 2

### 4-(2-méthylsulfonyloxy prop-2-yl)5-[(3-phénoxyphényl)-méthoxy]tétrahydrofuran-2-one (trans dl)

### Stade A

### 4-(2-méthyl sulfinyloxy prop-2-yl) 5-[(3-phénoxyphényl)-méthoxy]tétrahydrofuran-2-one (trans dl)

Dans un mélange de 10 cm³ de chlorure de méthylène et de 3 cm³ de pyridine, on introduit 3,08 g de 4-(2-hydroxy prop-2-yl) 5-[(3-phénoxyphényl)méthoxy] tétrahydro furan-2-one (trans dl), refroidit le mélange réactionnel à 0° C, introduit progressivement une solution de 0,89 g de chlorure de méthane sulfinyle dans 5 cm³ de chlorure de méthylène, agite pendant une heure à 0° C, lave à l'acide chlorhydrique N puis à l'eau, sèche, concentre à sec sous pression réduite et obtient 3,7 g de 4-(2-méthyl sulfinyloxy prop-2-yl) 5-[(3-phénoxyphényl)méthoxy]tétrahydrofuran-2-one (trans dl) brut.

Spectre I.R. (Chloroforme)
Absorption à 1781 $cm^{-1}$ caractéristique du carbonyle de la $\gamma$ lactone,
Absorptions à 1585 et 1489 $cm^{-1}$, caractéristiques du méta phénoxy phényle,
absorption à 1395 $cm^{-1}$, caractéristique des méthyles portés par le carbone en $\alpha$ de l'oxygène du sulfinate,
Absorption à 1119 $cm^{-1}$, caractéristique de $S \rightarrow O$
absorption à 694 $cm^{-1}$, caractéristique du noyau phényle.

Spectre de RMN (deutérochloroforme)

Pics à 1,40 – 1,42 – 1,50 ppm attribués aux hydrogènes des méthyles portés par le carbone en $\alpha$ de l'oxygène du groupement sulfinyle,
pics à 2,17 – 2,92 ppm attribués aux hydrogènes en positions 3 et 4 du cycle furanone
pics à 2,55 – 2,58 ppm attribués au méthyle du groupement méthane sulfinyle,
pics à 4,48 – 4,7 et 4,78 – 4,98 ppm attribués au méthylène en $\alpha$ du méta phénoxy phényle – quadruplet à 284 nm (J = 12,5 Hz),
pics à 5,38 – 5,42 et 5,51 – 5,55 ppm caractéristiques de l'hydrogène en position 5 de la furanone, quadruplet à 5,63 ppm (J = 2,5 Hz).

### Stade B

### 4-(2-methyl sulfonyloxy prop-2-yl)5-[(3-phénoxy phényl)-méthoxy]tétrahydrofuran-2-one (trans dl)

Dans 10 cm³ de chlorure de méthylène, on introduit 0,627 g de sulfinate abtenu au stade A de l'exemple 2, ajoute 0,430 g d'acide para nitro perbenzoïque, agite pendant une heure et quarante minutes à 20° C, elimine par filtration le précipité formé, lave le filtrat à l'eau, le sèche, filtre et obtient le 4-(2-méthyl sulfonyloxy oeio-2-yl)5-[(3-phénoxy-phényl)-méthoxy] tétrahydrofuran-2-one (trans dl) en solution chlorométhylénique.

Des contrôles effectués sur un échantillon concentré à sec montre que le produit obtenu possède des caractéristiques physiques analogues à celui de l'exemple 1.

## Exemple 3

5[(3-phénoxyphényl) méthoxy] 4(2-éthyl-sulfonyloxy prop-2-yl)-tétrahydrofuran-2-one (trans dl)

### Stade A

5-[(3-phénoxyphényl) méthoxy] 4(2-éthyl-sulfinyloxy-prop-2-yl)-tétrahydrofuran-2-one (trans dl)

Dans un mélange de 10 $cm^3$ de chlorure de méthylène et de 1 $cm^3$ de pyridine on introduit 2 g de 4-(2-hydroxy prop-2-yl) 5-[(3-phénoxyphényl) méthoxy] tétrahydrofuran-2-one (trans dl) refroidit à $-10°$ C, introduit progressivement 1 g de chlorure d'éthane sulfinyle en solution dans 5 $cm^3$ de chlorure de méthylène, laisse pendant 2 heures à 0° C, verse le mélange réactionnel sur une solution aqueuse diluée d'acide chlorhydrique, lave à l'eau, sèche, concentre à sec sous pression réduite et obtient 2,57 g de 5-[(3-phénoxyphényl) méthoxy] 4(2-éthyl-sulfinyloxy-prop-2-yl) tétrahydrofuran-2-one (trans dl).

Spectre IR (chloroforme)
- Absorption à 1783 $cm^{-1}$ caractéristique du carbonyle lactonique,
- Absorption à 1113 $cm^{-1}$ caractéristique de $S \rightarrow O$

Spectre de R.M.N. (deutérochloroforme)
- pics à 1,07 – 1,18 – 1,3 ppm attribués aux hydrogènes du méthyle de l'éthyle du groupement éthylsulfinyle.
- pics à 1,4 – 1,45 – 1,52 ppm attribués aux hydrogènes des méthyles portés par le carbone en $\alpha$ de l'oxygène du groupement sulfinyle.
- pics à 2,52 – 2,92 ppm attribués au méthylène de l'éthyle du groupement éthylsulfinyle.
- pics à 4,48 – 4,68 – 4,78 – 4,9 ppm attribués au méthylène du phénylméthoxy en position 5 de la furanone.
- pics à 5,48 – 5,52 – 5,62 – 5,66 ppm attribués à l'hydrogène en position 5 de la furanone.
- pics à 6,92 – 7,58 ppm attribués au noyau aromatique.

### Stade B

5[(3-phénoxyphényl)methoxy] 4-(2-éthyl sulfonyloxy-prop-2-yl)-tétrahydrofuran-2-one (trans dl)

A une suspension de 0,44 g de sulfinate obtenu au stade A de l'exemple 3, dans 4,5 $cm^3$ de chlorure de méthylène, on ajoute 0,50 g d'acétate de potassium anhydre ajoute progressivement, en 2 heures environ 1,6 $cm^3$ de solution d'acide peracétique (comprenant 40,5% d'acide acétique, 31% d'acide peracétique et 17,3% d'eau oxygénée), verse le mélange réactionnel lentement, à 0° C, sur une solution aqueuse de thiosulfate de sodium, extrait au chlorure de méthylène, lave la phase organique avec une solution de bicarbonate de sodium puis à l'eau, sèche, concentre à sec sous pression réduite, chromatographie le résidu sur gel de silice en éluant avec un mélange de benzène et d'acétate d'éthyle (8 – 2) et recueille 0,33 g de 5-(3-phénoxyphénylméthoxy) 4-(2-éthylsulfonyloxy prop-2-yl)-tétrahydrofuran-2-one, qui est conservé en solution dans un mélange de chlorure de méthylène et de benzène, à basse température, dont les spectres IR et RMN sont identiques à ceux du composé obtenu à l'exemple 1.

## Exemple 4

5[(3-phénoxyphényl) méthoxy] 4-(2-éthyl sulfonyloxy prop-2-yl)-tétrahydrofuran-2-one (trans dl)

### Stade A

On obtient le 5[(3-phénoxyphényl) méthoxy] 4-(2-éthyl sulfinyloxy-prop-2-yl) tétrahydrofuran-2-one (trans dl) comme au stade A de l'exemple 3.

Stade B

5[(3-phénoxyphényl) méthoxy] 4-(2-éthyl sulfonyloxy prop-2-yl)-tétrahydrofuran-2-one (trans dl)

A un mélange de 0,214 g de sulfinate obtenu au stade A de l'exemple 4, de 4 $cm^3$ d'acétone, de 0,3 $cm^3$ d'acide acétique et de 2 $cm^3$ d'eau on ajoute à 0°C, 0,084 g de permanganate de potassium, agite pendant 2 heures à 0°C, ajoute une solution de 0,050 g de permanganate de potassium dans 1 $cm^3$ d'eau et 1 $cm^3$ d'acétone, agite pendant 17 heures à 0°C, verse le mélange réactionnel sur une solution aqueuse de thiosulfate de sodium, extrait au chlorure de méthylène, lave la phase organique avec une solution de bicarbonate de sodium, puis à l'eau, sèche, concentre à sec sans chauffer, purifie le résidu par chromatographie sur gel de silice en éluant par un mélange de benzène et d'acétate d'éthyle (8–2) et obtient 0,090 g de 5[(3-phénoxyphényl) méthoxy] 4-(2-éthyl sulfonyloxy prop-2-yl)-tétrahydrofuran-2-one (trans dl) dont les spectres IR et RMN sont identiques à ceux du composé abtenu à l'exemple 1.

Exemple 5

4-[2-(prop-2-yl sulfonyloxy) prop-2-yl] 5-[(3-phénoxyphényl)-méthoxy] tétrahydrofuran-2-one (trans dl)

Stade A

4-[2-(prop-2-yl sulfinyloxy) prop-2-yl] 5-[(3-phénoxyphényl)-méthoxy] tétrahydrofuran-2-one (trans dl)

Dans un mélange de 10 $cm^3$ de chlorure de méthylène et de 3 $cm^3$ de pyridine, on introduit 3,25 g d'alcool 4-(2-hydroxy prop-2-yl) 5-[(3-phénoxyphényl)-méthoxy] tétrahydrofuran-2-one (trans dl), amène le mélange réactionnel à 0°C, ajoute progressivement une solution de 1,2 g de chlorure d'isopropane sulfinyle dans 5 $cm^3$ de chlorure de méthylène, agite pendant 17 heures à 20°C, lave à l'acide chlorhydrique puis à l'eau, concentre à sec par distillation sous pression réduite et obtient 4,2 g de 4-[2-(prop-2-yl sulfinyloxy) prop-2-yl] 5-[(3-phénoxyphényl) méthoxy] tétrahydrofuran-2-one (trans dl).

Spectre IR (chloroforme)

Absorption à 1780 $cm^{-1}$ due au carbonyle de la $\gamma$ lactone.
Absorption à 1583, 1486 $cm^{-1}$ due au groupement métaphénoxyphényle.
Absorption à 1392 et 1379 $cm^{-1}$ due aux méthyles géminés.
Absorption à 1110 $cm^{-1}$ due à

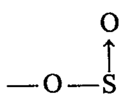

Spectre de RMN (deutérochloroforme)

pics à 1,08–1,52 ppm attribués aux hydrogènes des méthyles de l'isopropyle.
pics à 2,0–3,0 ppm attribués aux hydrogènes en position 3 et 4 de la furanone ainsi qu'a l'hydrogène porté par le carbone en $\alpha$ de l'atome de soufre.
pics à 4,45–4,65 et 4,75–4,95 ppm attribués aux hydrogènes du méthylène en $\alpha$ du métaphénoxyphényle quadruplet à 282 ppm (J = 12 Hz).
pics à 5,44–5,48 et 5,57–5,61 ppm attribués à l'hydrogène en position 5 de la furanone – quadruplet à 331,5 ppm (J = 2,5 Hz).

Stade B

4-[2-(prop-2-yl sulfonyloxy) prop-2-yl] 5-[(3-phénoxyphényl)-méthoxy] tétrahydrofuran-2-one (trans dl)

Dans 10 $cm^3$ de chlorure de méthylène on introduit 0,404 g de sulfinate obtenu au stade A de l'exemple 5,ajoute 0,287 g d'acide para nitroperbenzoïque (titrant 80%), agite pendant 50 minutes à 20°C, élimine par filtration le précipité formé, lave le filtrat par une solution aqueuse de bicarbonate de sodium, à l'eau, concentre à sec par distillation sous pression réduite et obtient 0,520 g de

4-[2-(prop-2-yl sulfonyloxy) prop-2-yl] 5-[(3-phénoxyphényl) méthoxy] tétrahydrofuran-2-one (trans dl).

Spectre IR (chloroforme)
Absorption à 1782 $cm^{-1}$ caractéristique du $-C=0$ de la $\gamma$ lactone.
Absorption à 1583, 1485 $cm^{-1}$ caractéristique du groupement métaphénoxyphényle.
Absorption à 1330 $cm^{-1}$ et 1152 – 1160 $cm^{-1}$ caractéristique de $-O-SO_2$.

## Exemple 6

### 4-[2-(paratolylsulfonyloxy) pro-2-yl] 5-[(3-phénoxyphényl)méthoxy]-tétrahydrofuran-2-one (trans dl)

### Stade A

### 4-[2-(paratolylsulfinyloxy) prop-2-yl] 5-[(3-phénoxyphényl)-méthoxy] tétrahydrofuran-2-one (trans dl)

Dans 20 $cm^3$ de chlorure de méthylène on introduit 1,5 g de 5[(3-phénoxyphényl) méthoxy] 4(2-hydroxy prop-2-yl) tétrahydrofuran-2-one (trans dl), 0,802 g de chlorure de paratoluène sulfinyle, ajoute, à 0° C, 10 $cm^3$ de pyridine, agite pendant 3 heures à 0° C, verse le mélange réactionnel dans une solution aqueuse N d'acide chlorhydrique, extrait au benzène, lave par une solution aqueuse d'acide chlorhydrique, par une solution aqueuse de bicarbonate de sodium, concentre à sec par distillation sous pression réduite, chromatographie le résidu sur gel de silice en éluant par un mélange de benzène et d'acétate d'éthyle (8 – 2) et obtient le 4-[2-(paratolylsulfinyloxy) prop-2-yl] 5-[(3-phénoxy-phényl)méthoxy] tétrahydrofuran-2-one (trans dl).

Spectre IR (chloroforme)
Absorption à 1783 $cm^{-1}$ caractéristique du carbonyle de la $\gamma$ lactone.
Absorption à 1585 $cm^{-1}$ et 1488 $cm^{-1}$ caractéristique du métaphénoxyphényle.
Absorption à 1393 $cm^{-1}$ caractéristique des méthyles géminés.
Absorption à 1110 – 1145 $cm^{-1}$ caractéristique de $S \rightarrow O$.

Spectre de RMN (deutérochloroforme)
pics à 1,50 – 1,58 ppm attribués aux hydrogènes des méthyles géminés.
pic à 2,38 ppm attribué à l'hydrogène du méthyle du paratolyle.
pics à 2,57 – 2,62 ppm attribués aux hydrogènes en position 4 et 5 du noyau tétrahydrofuran.
pics à 4,41 – 5,0 ppm attribués aux hydrogènes du méthylène en $\alpha$ du métaphénoxyphényle.
pics à 5,4 – 5,53 ppm attribués à l'hydrogènes en position 3 du cycle tétrahydrofuranyle (2 stéréoisomères).
pics à 6,83 – 7,50 ppm attribués aux noyaux aromatiques.

### Stade B

### 4-[2-(paratolylsulfonyloxy) prop-2-yl] 5-[(3-phénoxyphényl)méthoxy]-tétrahydrofuran-2-one (trans dl)

Dans 10 $cm^3$ de chloroforme on introduit 0,420 g de dérivé sulfinylé obtenu au stade A de l'exemple 6, ajoute 0,320 g d'acide para nitroperbenzoïque (titrant 80%), agite pendant 3 heures et 30 minutes, à 20° C, refroidit à – 10° C, élimine par filtration l'insoluble formé, concentre le filtrat par destillation sous pression réduite, effectue une nouvelle filtration, concentre à sec par destillation sous pression réduite et obtient le 4-[2-(paratolylsulfonyloxy) prop-2-yl] 5-[(3-phénoxyphényl)methoxy] tétrahydrofuran-2-one (trans dl).

Spectre IR (chloroforme)
Absorption à 1783 $cm^{-1}$ attribuée au carbonyle de la $\gamma$ lactone.
Absorption à 1350 $cm^{-1}$, 1175 $cm^{-1}$ attribuée au $-SO_2$.

## Exemple 7

6,6-diméthyl 4-[(3-phénoxyphényl)méthoxy] 3-oxabicyclo [3,1,0] hexan-2-one

A une solution de 0,30 g de 4-(2-éthyl sulfonyloxy prop-2-yl) 5-[(3-phénoxyphényl)méthoxy]tétrahydrofuran-2-one (trans dl) dans 3 cm³ de chlorure de méthylène on ajoute, à 20° C, 2 cm³ de solution aqueuse de soude à 50 g pour 100 g puis 0,015 g de chlorure de triéthylbenzyl ammonium, agite pendant 25 minutes à 20° C, verse le mélange réactionnel sur une solution aqueuse glacée de phosphate monosidique, extrait au chlorure de méthylène, concentre à sec par distillation sous pression réduite, purifie le résidu par chromatographie sur gel de silice en éluant avec un mélange d'éther de pétrole 35—75° C et d'éther éthylique (7—3) et obtient 0,140 g de 6,6-diméthyl-4-[(3-phénoxyphényl)methoxy]3-one bicyclo [3,1,0] hexan-2-one, F = 66° C.

## Exemple 8

(4S, 5R) 5[(1R, 2S, 5R) 2-prop-2-yl 5-méthylcyclohexyloxy] 4(2-méthylsulfonyloxy-prop-2-yl)-tétrahydrofuran-2-one

A une solution de 1,5 g de (4S, 5R) (−) 5[(1R, 2S, 5R) 2-prop-2-yl 5-methyl cyclohexyloxy] 4(2-hydroxy-prop-2-yl) tétrahydrofuran-2-one dans 30 cm³ de benzène, on ajoute à +5° C, 1,5 cm³ de triéthylamine, introduit très lentement à 5° C, 0,8 cm³ de chlorure de méthane sulfonyle en solution dans 6 cm³ de benzène, verse le mélange réactionnel sur de l'eau glacée, sépare par décantation la phase organique, la lave par une solution aqueuse de phosphate monosodique de bicarbonate de sodium, à l'eau, la sèche, la concentre à sec sous pression réduite et dilue le résidu dans le chlorure de méthylène. La solution chlorométhylénique est conservée à 0° C.

Spectre IR (chloroforme)

Absorption à 1780 $cm^{-1}$ due au carbonyle de la lactone.

Absorption à 1339 — 1178 $cm^{-1}$ due à

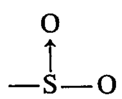

Spectre de RMN (deutérochloroforme)

Pics à 0,73 — 0,75 — 0,83 — 0,95 ppm attribués aux hydrogènes des méthyles de l'isopropyle en position 2 du cyclohexyle.

Pics à 0,88 — 0,98 ppm attribués aux hydrogènes du méthyle en position 5 du cyclohexyle.

Pics à 1,65 — 1,72 ppm attribués aux hydrogènes des méthyles portés par le carbone en $\alpha$ de l'oxygène du groupement sulfonyle.

La (4S, 5R) (−) 5 [(1R, 2S, 5R) 2-prop-2-yl 5-méthyl cyclohexyloxy]4-(2-hydroxy prop-2-yl) tétrahydrofuran-2-one utilisée au départ de l'exemple 8 peut être préparée de la manière qui suit:

### Stade A

(5R) (−)-[(1R, 2S, 5R) 2-prop-2-yl 5-méthyl cyclohexyloxy] (5H) furan-2-one

On chauffe au reflux 32,5 g de l menthol, 21 g de 5-hydroxy 2,5-dihydro furan-2-one, 0,2 g d'acide paratoluène sulfonique et 300 cm³ de benzène, en éliminant l'eau formée par entrainement azéotropique. On ajoute 4 g de 5-hydroxy 2,5-dihydro furan-2-one, maintient au reflux pendant 1 heure, refroidit, lave la solution organique au bicarbonate de sodium, puis à l'eau, sèche, concentre à sec sous pression réduite, dilue le résidu obtenu (51,6 g) dans 100 cm³ d'éther de pétrole (eb. 35 — 70° C), laisse pendant 17 heures à 0° C, isole par essorage les cristaux formés et obtient 15 g de produit attendu (F = 76° C).

On concentre les liqueurs mères à sec, dilue le résidu dans 150 cm³ de benzène, ajoute 200 mg d'acide paratoluène sulfonique, porte le mélange réactionel au reflux 3 heures, refroidit, lave avec une solution aqueuse de bicarbonate de sodium à l'eau, sèche, concentre à sec, cristallise le résidu dans l'éther de pétrole (eb. 35 — 70° C) et obtient 10, 35 g de produit attendu, F = 76° C.

De nouveau on fait subir aux liqueurs mères un traitement analogue à celui décrit ci-dessus et obtient 5,7 g de produit attendu F = 76° C.

Un dernier traitement analogue fournit 3,4 g de produit attendu F = 76° C.

En résumé on a obtenu 34,45 g de produit attendu pur F = 76° C.

Le (5R) (−) 5-[(1R, 2S, 5R) 2-propyl-2-yl 5-méthyl cyclohexyloxy] (5H) furan-2-one ainsi obtenu posséde les charactéristiques suivantes:

$[\alpha]_D = -139°$ (c = 1,5%, chloroforme)

Spectre RMN (deutérochloroforme)
pics à 0,75−0,86 ppm; 0,86−1,0 ppm; 0,83−0,94 ppm; attribués aux hydrogènes du méthyle en position 6 du radical menthyle et des hydrogènes des méthyles de l'isopropyle en position 2 du radical menthyle.
pic à 3,66 ppm attribué à l'hydrogène en position 1 du radical menthyle.
pics à 6,10−6,12−6,13 ppm attribués aux hydrogènes en position 5 du radical furanone.
pics à 6,16−6,18; 6,25−6,26 ppm attribués à l'hydrogène en position 3 du radical furanone.
pics à 7,13−7,15; 7,21−7,23 ppm attribués à l'hydrogène en position 4 du radical furanone.

Stade B

(4S, 5R) (−) [(1R, 2S, 5R) 2-prop-2-yl 5-méthyl cyclohexyloxy] 4-(2-hydroxy-prop-2-yl)-tétrahydrofuran-2-one

On dissout 5 g de (5R) (−) [(1R, 2S, 5R) 2-prop-2-yl 5-méthyl cyclohexyloxy] (5H) furan-2-one obtenu au stade A précédent dans 100 $cm^3$ d'isopropanol, ajoute 0,125 g de carbonate de calcium, porte le mélange au reflux, maintient le reflux pendant 5 minutes sous atmosphère inerte, ajoute 0,200 g de peroxyde de benzoyle, porte le mélange réactionnel au reflux, l'y maintient pendant 3 heures et 30 minutes, refroidit, filtre, concentre à sec sous pression réduite, agite le résidu avec de l'eau, isole, par essorage, les cristaux formés, purifie le résidu par chromatographie sur gel de silice en éluant avec un mélange de benzène et d'acétate d'éthyle (8−2), puis par cristallisation dans l'éther de pétrole et obtient le (4S, 5R), (−) 5-[(1R, 2S, 5R) 2-prop-2-yl 5-méthyl cyclohexyloxy] 4-(2-hydroxy-prop-2-yl) tétrahydrofuran-2-one F = 89° C,

$[\alpha]_D = -154,5° + 2,5°$ (c = 1,5%, chloroforme)

Spectre IR (chloroforme)
Absorption à 3608 $cm^{-1}$ due au groupement hydroxy,
Absorption à 1777 $cm^{-1}$ due au carbonyle de la $\gamma$ lactone.

Spectre RMN (deutérochloroforme)
pics à 0,73−0,85−0,82−0,93 ppm attribués aux hydrogènes des méthyles de l'isopropyle en position 2 du cyclohexyle,
pics à 0,88−0,96 ppm attribués aux hydrogènes du méthyle en position 5 du cyclohexyle,
pics à 1,22−1,28 ppm caractéristiques des hydrogènes des méthyles portés par le carbone en $\alpha$ de l'hydroxy,
pics à 5,67−5,72 ppm caractéristiques de l'hydrogène en position 5 de la furanone.

Exemple 9

(1R, 4R, 5S) (−) 6,6-diméthyl 4−[(1R, 2S, 5R) 2-prop-2-yl 5-méthyl, cyclohexyloxy] 3-oxa bicyclo [3,1,0] hexan-2-one

On refroidit la solution chlorométhylénique obtenue à l'exemple 8 à +5° C, ajoute 5 $cm^3$ de solution aqueuse de soude à 50%, maintient la température à 10−15°C, ajoute 0,050 g de chlorure de triéthylbenzylammonium, maintient la température réactionnelle à 12° ±2° C pendant 2 heures et 30 minutes en agitant rapidement. On verse lentement le mélange réactionnel sur une solution aqueuse de phosphate monosodique, extrait au chlorure de méthylène, lave la phase organique à l'eau, sèche, concentre à sec en terminant sous pression réduite, chromatographie le résidu sur gel de silice en éluant par un mélange d'éther de pétrole et d'éther éthylique (8−2) et obtient 1,18 g de (1R, 4R, 5S) (−) 6,6-diméthyl 4-[(1R, 2S, 5R) 2-prop-2-yl 5-méthyl cyclohexyloxy] 3-oxa bicyclo [3,1,0] hexan-2-one F = 82° C,

$[\alpha]_D^{20} = -195° \pm 2$ (c = 4%, chloroforme).

Ce produit est identique en tout point à celui obtenu par condensation du 1-menthol et de la (1R, 4R, 5S) (−) 6,6-diméthyl 4-hydroxy 3-oxa bicyclo [3,1,0] hexan-2-one.

Analyse: $C_{17}H_{28}O_3$ (280,42)
Calculé: C 72,81 H 10,06%
Trouvé: C 72,9 H 10,0%

De façon analogue à celle de l'exemple 8 du départ de 4-[2-(prop-2-yl sulfonyloxy) prop-2-yl] 5-[(3-phénoxyphényl) méthoxy] tétrahydrofuran-2-one, en utilisant la soude aqueuse dans le chlorure de méthylène et le chlorure de triéthyl benzyl ammonium, on obtient le (1R, 4R, 5S) 6,6-diméthyl 4-[(3-phénoxyphényl) méthoxy] 3-oxa bicyclo [3,1,0] hexan-2-one.

Exemple 10

(1R, 4R, 5S) (−) 6,6-diméthyl, 4-hydroxy 3-oxa bicyclo [3,1,0] hexan-2-one

A une solution de 0,805 g de dérivé menthylé obtenu à l'exemple 9 (F = 82° C) dans 8 $cm^3$ d'acétone, on ajoute lentement 8 $cm^3$ de solution aqueuse 0,5 N d'acide chlorhydrique, agite pendant 4 heures à 20° C, extrait la phase aqueuse à l'éther de pétrole, concentre la phase aqueuse à sec sous pression réduite et obtient 0,360 g de (1R, 4R, 5S) (−) 6,6-diméthyl, 4-hydroxy 3-oxa bicyclo [3,1,0] hexan-2-one, F = 116° C,

$[\alpha]_D = -114° \pm 15°$ (c = 1%, diméthylformamide).

**Revendications pour les Etats contractants: BE, CH, DE, GB, IT, LI, NL, SE**

1. Sous forme trans en positions 4 et 5, de configuration (4SR, 5RS), (4S, 5R) ou (4R, 5S), les composés de formule générale ($I_A$):

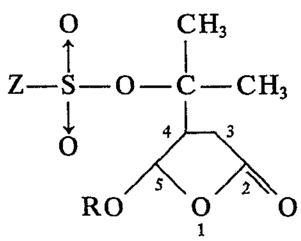

($I_A$)

dans laquelle Z représente un radial alcoyle comportant de 1 à 4 atomes de carbone, un radical aryle monocyclique éventuellement substitué soit par un groupement alcoyle comportant de 1 à 4 atomes de carbone, soit par un groupement alcoxyle comportant de 1 à 4 atomes de carbone, soit par un atome d'halogène, soit par un groupement nitro et R représente le reste organique du menthol ou de l'alcool méta-phenoxybenzylique.

2. Procédé de préparation des dérivés cyclopropaniques de configuration (1RS, 4RS, 5SR), (1S, 4S, 5R) ou (1R, 4R, 5S) de formule générale (V):

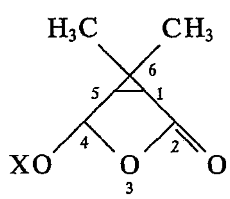

(V)

dans laquelle X représente un atome d'hydrogène ou le reste R d'un alcool R—OH éventuellement chiral, caractérisé en ce que l'on fait réagir, un composé de formule générale (II):

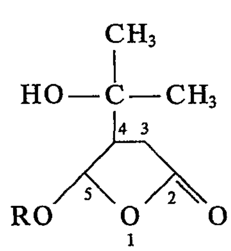

(II)

dans laquelle R représente le reste organique d'un alcool R—OH éventuellement chiral, le composé (II) étant de structure trans en position 4 et 5, et ayant dans le cas où R est le reste d'un alcool achiral une configuration (4 SR, 5 RS) et dans le cas où R est le reste d'un alcool chiral une configuration (4 S, 5 R) ou (4 R, 5 S), avec un composé de formule (III)

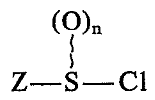

(III)

dans laquelle n = 1 ou n = 2, le treit ondulé représente une ou deux liaisons semi-polaires liant le ou les atomes d'oxygène à l'atome de soufre, suivant que n = 1 ou n = 2, et Z représente un radical alcoyle comportant de 1 à 4 atomes de carbone, un radical aryle monocyclique éventuellement substitué soit par un groupement alcoyle comportant de 1 à 4 atomes de carbone, soit par un groupement alcoxyle comportant de 1 à 4 atomes de carbone, soit par un atome d'halogène, soit par un groupement nitro,
— soit pour obtenir lorsque n = 1, un composé de structure trans en positions 4 et 5, de formule générale (IV):

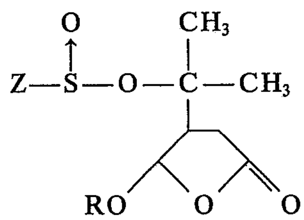

(IV)

dans laquelle R et Z conservent les significations précitées, que l'on fait réagir avec un agent oxydant, pour obtenir un composé de formule générale (I):

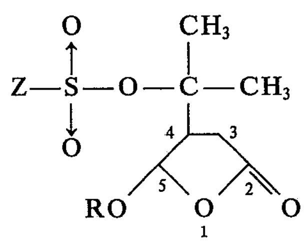

(I)

de structure trans en positions 4 et 5, de configuration (4SR, 5RS) si le reste d'alcool R est achiral ou de configuration (4S, 5R) ou (4R, 5S) si le reste d'alcool R est chiral,
— soit pour obtenir lorsque n = 2, un composé de formule générale (I) dans laquelle R et Z conservent les significations précitées, le composé de formule générale (I) étant de configuration (4SR, 5RS) si le reste d'alcool R est achiral et de configuration (4S, 5R) ou (4R, 5S) si le reste R est chiral, puis soument à l'action d'un agent basique, un composé de formule générale (I), la cyclisation résultante ayant lieu avec rétention de configuration en ce qui concerne les carbones en positions 4 et 5 et faisant correspondre un composé V de configuration (1RS, 4RS, 5SR) à un composé I de configuration (4SR, 5RS) un composé V de configuration (1R, 4R, 5S) à un composé I de configuration (4S, 5R) et un composé V de configuration (1S, 4S, 5R) à un composé I de configuration (4R, 5S), puis hydrolyse, si désiré, en milieu acide, avec rétention de configuration, la fonction éther du composé V dans lequel X est un reste d'alcool.

3. Procédé selon la revendication 2, caractérisé en ce que, lorsque n = 1, l'on opère la condensation du chlorure de sulfinyle III et du composé II, en présence d'une base tertiaire et au sein d'un solvant choisi dans le groupe constitué par les hydrocarbures halogénés et les hydrocarbures aromatiques.

4. Procédé selon la revendication 3, caractérisé en ce que la base tertiaire est la pyridine et le solvant est le chlorure de méthylène.

5. Procédé selon la revendication 2, caractérisé en ce que l'agent oxydant est le permanganate de potassium, l'eau oxygénée, l'acide peracétique ou l'acide paranitroperbenzoïque.

6. Procédé selon l'une quelconque des revendications 2 et 5, caractérisé en ce que l'action de l'agent oxydant est effectuée au sein d'un solvant ou d'un mélange de solvants choisi dans le groupe constitué par les hydrocarbures halogénés, les hydrocarbures aromatiques, l'acide acétique, l'acétone, les alcanols et l'eau.

7. Procédé selon la revendication 2, caractérisé en ce que, lorsque n = 2, l'on opère la condensation ou chlorure de sulfonyle III et du composé II en présence de triéthylamine et au sein d'un solvant choisi dans le groupe constitué par les hydrocarbures halogénés et les hydrocarbures aromatiques.

8. Procédé selon la revendication 7, caractérisé en ce que le solvant est le benzène.

9. Procédé selon la revendication 2, caractérisé en ce que l'agent basique auquel on soumet le composé de formule I est un hydroxyde alcalin.

10. Procédé selon la revendication 2 ou 9, caractérisé en ce que l'agent basique est utilisé selon la méthode dite de catalyse par transfert de phase.

11. Procédé selon la revendication 10, caractérisé en ce que, dans la méthode dite de catalyse par transfert de phase, le solvant est le chlorure de méthylène, le catalyseur est le chlorure de triéthylbenzylammonium, l'agent basique est la soude et l'on opère en présence d'eau.

**Revendications pour l'Etat contractant: AT**

1. Procédé de préparation des dérivés cyclopropaniques de configuration (1RS, 4RS, 5SR), (1S, 4S, 5R) ou (1R, 4R, 5S) de formule générale (V):

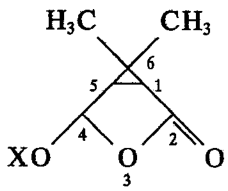

(V)

dans laquelle X représente un atome d'hydrogène ou le reste R d'un alcool R—OH éventuellement chiral, caractérisé en ce que l'on fait réagir, un composé de formule générale (II):

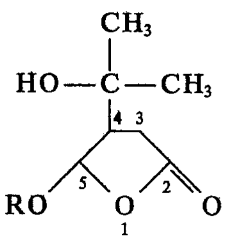

(II)

dans laquelle R représente le reste organique d'un alcool R—OH éventuellement chiral, le composé (II) étant de structure trans en position 4 et 5, et ayant dans le cas où R est le reste d'un alcool achiral une configuration (4 SR, 5 RS) et dans le cas où R est le reste d'un alcool chiral une configuration (4 S, 5 R) ou (4 R, 5 S), avec un composé de formule (III)

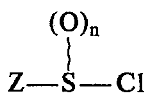

(III)

dans laquelle n=1 ou n=2, le trait ondulé représente une ou deux liaisons semi-polaires liant le ou les atomes d'oxygène à l'atome de soufre, suivant que n=1 ou n=2, et Z représente un radical alcoyle comportant de 1 à 4 atomes de carbone, un radical aryle monocyclique éventuellement substitué soit par un groupement alcoyle comportant de 1 à 4 atomes de carbone, soit par un groupement alcoxyle comportant de 1 à 4 atomes de carbone, soit par un atome d'halogène, soit par un groupement nitro,

— soit pour obtenir lorsque n=1, un composé de structure trans en positions 4 et 5, de formule générale (IV):

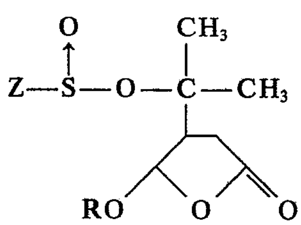

(IV)

dans laquelle R et Z conservent les significations précitées, que l'on fait réagir avec un agent oxydant, pour obtenir un composé de formule générale (I):

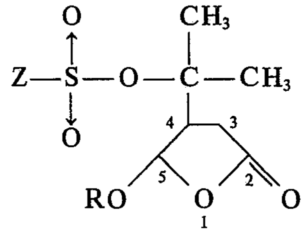

(I)

de structure trans en positions 4 et 5, de configuration (4SR, 5RS) si le rest d'alcool R est achiral ou de configuration (4S, 5R) ou (4R, 5S) si le reste d'alcool R est chiral,

– soit pour obtenir lorsque n = 2, un composé de formule générale (I) dans laquelle R et Z conservent les significations précédentes, le composé de formule générale (I) étant de configuration (4SR, 5RS) si le reste d'alcool R est achiral et de configuration (4S, 5R) ou (4R, 5S) si le reste R est chiral, puis soument à l'action d'un agent basique, un composé de formule générale (I), la cyclisation résultante ayant lieu avec rétention de configuration en ce qui concerne les carbones en positions 4 et 5 et faisant correspondre un composé V de configuration (1RS, 4RS, 5SR) à un composé I de configuration (4SR, 5RS) un composé V de configuration (1R, 4R, 5S) à un composé I de configuration (4S, 5R) et un composé V de configuration (1S, 4S, 5R) à un composé I de configuration (4R, 5S), puis hydrolyse, si désiré, en milieu acide, avec rétention de configuration, la fonction éther du composé V dans lequel X est un reste d'alcool.

2. Procédé selon la revendication 1, caractérisé en ce que, lorsque n = 1, l'on opère la condensation du chlorure de sulfinyle III et du composé II, en présence d'une base tertiaire et au sein d'un solvant choisi dans le groupe constitué par les hydrocarbures halogénés et les hydrocarbures aromatiques.

3. Procédé selon la revendication 2, caractérisé en ce que la base tertiaire est la pyridine et le solvant est le chlorure de méthylène.

4. Procédé selon la revendication 1, caractérisé an ce que l'agent oxydant est le permanganate de potassium, l'eau oxygénée, l'acide peracétique ou l'acide paranitroperbenzoïque.

5. Procédé selon l'une quelconque des revendications 1 et 4, caractérisé en ce que l'action de l'agent oxydant est effectuée au sein d'un solvant ou d'un mélange de solvants choisi dans le groupe constitué par les hydrocarbures halogénés, les hydrocarbures aromatiques, l'acide acétique, l'acétone, les alcanols et l'eau.

6. Procédé selon la revendication 1, caractérisé en ce que, lorsque n = 2, l'on opère la condensation du chlorure de sulfonyle III et du composé II en présence de triéthylamine et au sein d'un solvant choisi dans le groupe constitué par les hydrocarbures halogénés et les hydrocarbures aromatiques.

7. Procédé selon la revendication 6, caractérisé en ce que le solvant est le benzène.

8. Procédé selon la revendication 1, caractérisé en ce que l'agent basique auquel on soumet le composé de formule I est un hydroxyde alcalin.

9. Procédé selon la revendication 1 ou 8, caractérisé en ce que l'agent basique est utilisé selon la méthode dite de catalyse par transfert de phase.

10. Procédé selon la revendication 9, caractérisé en ce que, dans la méthode dite de catalyse par transfert de phase, le solvant est le chlorure de méthylène, le catalyseur est le chlorure de triéthylbenzylammonium, l'agent basique est la soude et l'on opère en présence d'eau.

**Patentansprüche für die Vertragsstaaten BE, CH, DE, GB, IT, LI, NL, SE**

1. In trans-Form im Hinblick auf die 4- und 5-Positionen mit der Konfiguration (4SR, 5RS), (4S, 5R) oder (4R, 5S), die Verbindungen der allgemeinen Formel ($I_A$):

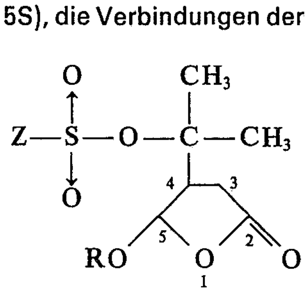

($I_A$)

worin Z einen Alkylrest mit 1 bis 4 Kohlenstoffatomen, einen monocyclischen Arylrest, der gegebenenfalls substituiert ist, entweder durch eine Alkylgruppe mit 1 bis 4 Kohlenstoffatomen oder durch eine Alkoxygruppe mit 1 bis 4 Kohlenstoffatomen oder durch ein Halogenatom oder durch eine Nitrogruppe bedeutet und R den organischen Rest von Menthol oder des m-Phenoxybenzylalkohols darstellt.

2. Verfahren zur Herstellung von Cyclopropanderivaten mit der Konfiguration (1RS, 4RS, 5SR), (1S,

4S, 5R) oder (1R, 4R, 5S) der allgemeinen Formel (V):

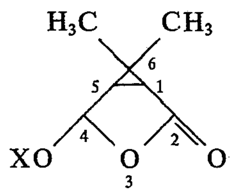

(V)

worin X ein Wasserstoffatom oder den Rest R eines gegebenenfalls chiralen Alkohols R—OH darstellt, dadurch gekennzeichnet, daß man eine Verbindung der allgemeinen Formel (II):

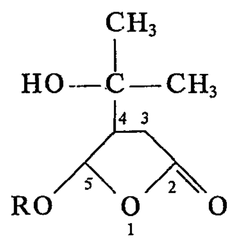

(II)

worin R den organischen Rest eines gegebenenfalls chiralen Alkohols R—OH darstellt, wobei die Verbindung (II) trans-Struktur im Hinblick auf die 4- und 5-Position besitzt und in dem Fall, in dem R den Rest eines achiralen Alkohols darstellt, die Konfiguration (4 SR, 5 RS) aufweist und in dem Fall, in dem R den Rest eines chiralen Alkohols darstellt, die Konfiguration (4 S, 5 R) oder (4 R, 5 S) besitzt, mit einer Verbindung der Formel (III)

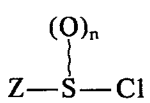

(III)

umsetzt, worin n = 1 oder n = 2, die gewellte Linie ein oder zwei semipolare Bindungen darstellt, die das Sauerstoffatom oder die Sauerstoffatome entsprechend n = 1 oder n = 2 mit dem Schwefelatom verbinden, und Z einen Alkylrest mit 1 bis 4 Kohlenstoffatomen, einen monocyclischen Arylrest, der gegebenenfalls substituiert ist, entweder durch eine Alkylgruppe mit 1 bis 4 Kohlenstoffatomen oder durch eine Alkoxygruppe mit 1 bis 4 Kohlenstoffatomen oder durch ein Halogenatom oder durch eine Nitrogruppe bedeutet,

entweder um, wenn n = 1, eine Verbindung mit trans-Struktur im Hinblick auf die Positionen 4 und 5 der allgemeinen Formel (IV)

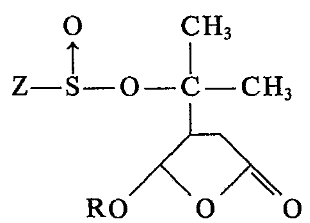

(IV)

zu erhalten, worin R und Z die vorstehend angegebenen Bedeutungen besitzen, welche man mit einem Oxidationsmittel umsetzt, um eine Verbindung der allgemeinen Formel (I)

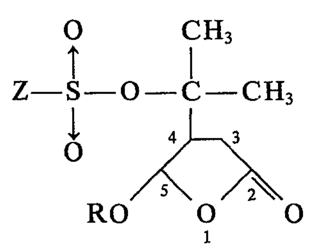

(I)

mit trans-Struktur im Hinblick auf die Positionen 4 und 5, mit der Konfiguration (4SR, 5RS), wenn der Rest des Alkohols R achiral ist oder der Konfiguration (4S, 5R) oder (4R, 5S), wenn der Rest des Alkohols R chiral ist, zu erhalten,

oder um, wenn n=2, eine Verbindung der allgemeinen Formel (I), worin R und Z die vorstehend angegebenen Bedeutungen besitzen, die Verbindung der allgemeinen Formel (I) mit der Konfiguration (4SR, 5RS), wenn der Rest des Alkohols R achiral ist und der Konfiguration (4S, 5R) oder (4R, 5S), wenn der Rest R chiral ist, zu erhalten, danach die Verbindung der allgemeinen Formel (I) der Einwirkung eines basischen Mittels unterzieht, wobei die resultierende Cyclisierung unter Retention der Konfiguration, was die Kohlenstoffatome in den Positionen 4 und 5 anbelangt, stattfindet und dazu führt, daß eine Verbindung V mit der Konfiguration (1RS, 4RS, 5SR) einer Verbindung I mit der Konfiguration (4SR, 5RS), eine Verbindung V mit der Konfiguration (1R, 4R, 5S) einer Verbindung I mit der Konfiguration (4S, 5R) und eine Verbindung V mit der Konfiguration (1S, 4S, 5R) einer Verbindung I mit der Konfiguration (4R, 5S) entspricht, danach gewünschtenfalls in saurem Milieu unter Retention der Konfiguration die Ätherfunktion der Verbindung V, worin X einen Rest des Alkohols darstellt, hydrolysiert.

3. Verfahren gemäß Anspruch 2, dadurch gekennzeichnet, daß, wenn n=1, man die Kondensation des Sulfinylchlorids III und der Verbindung II, in Gegenwart einer tertiären Base und in dem Medium eines Lösungsmittels, ausgewählt unter den halogenierten Kohlenwasserstoffen und den aromatischen Kohlenwasserstoffen, durchführt.

4. Verfahren gemäß Anspruch 3, dadurch gekennzeichnet, daß die tertiäre Base Pyridin ist und das Lösungsmittel Methylenchlorid ist.

5. Verfahren gemäß Anspruch 2, dadurch gekennzeichnet, daß das Oxidationsmittel Kaliumpermanganat, Wasserstoffperoxid, Peressigsäure oder p-Nitroperbenzoesäure ist.

6. Verfahren gemäß einem der Ansprüche 2 und 5, dadurch gekennzeichnet, daß die Umsetzung des Oxidationsmittels in dem Medium eines Lösungsmittels oder eines Gemisches von Lösungsmitteln, ausgewählt unter den halogenierten Kohlenwasserstoffen, den aromatischen Kohlenwasserstoffen, der Essigsäure, dem Aceton, den Alkanolen, und Wasser, durchgeführt wird.

7. Verfahren gemäß Anspruch 2, dadurch gekennzeichnet, daß, wenn n=2, die Kondensation des Sulfonylchlorids III und der Verbindung II in Gegenwart von Triäthylamin und in dem Medium eines Lösungsmittels, ausgewählt unter den halogenierten Kohlenwasserstoffen und den aromatischen Kohlenwasserstoffen, durchgeführt wird.

8. Verfahren gemäß Anspruch 7, dadurch gekennzeichnet, daß das Lösungsmittel Benzol ist.

9. Verfahren gemäß Anspruch 2, dadurch gekennzeichnet, daß das basische Mittel, mit dem man die Verbindung der Formel I umsetzt, ein Alkalihydroxid ist.

10. Verfahren gemäß Anspruch 2 oder 9, dadurch gekennzeichnet, daß das basische Mittel nach der als Phasentransferkatalyse bezeichneten Methode verwendet wird.

11. Verfahren gemäß Anspruch 10, dadurch gekennzeichnet, daß bei der als Phasentransferkatalyse bezeichneten Methode das Lösungsmittel Methylenchlorid, der Katalysator Triäthylbenzylammoniumchlorid, das basische Mittel Natronlauge ist und man in Gegenwart von Wasser arbeitet.

**Patentansprüche für den Vertragsstaat AT**

1. Verfahren zur Herstellung von Cyclopropanderivaten mit der Konfiguration (1RS, 4RS, 5SR), (1S, 4S, 5R) oder (1R, 4R, 5S) der allgemeinen Formel (V)

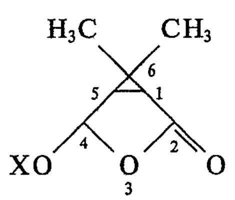

(V)

worin X ein Wasserstoffatom oder den Rest R eines gegebenenfalls chiralen Alkohols R—OH darstellt, dadurch gekennzeichnet, daß man eine Verbindung der allgemeinen Formel (II):

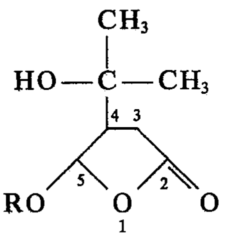

(II)

worin R den organischen Rest eines gegebenenfalls chiralen Alkohols R—OH darstellt, wobei die Verbindung (II) im Hinblick auf die Positionen 4 und 5 trans-Struktur besitzt und in dem Fall, in dem R den Rest eines achiralen Alkohols darstellt, die Konfiguration (4 SR, 5 RS) aufweist, und in dem Fall, in dem R

den Rest eines chiralen Alkohols darstellt, die Konfiguration (4 S, 5 R) oder (4 R, 5 S) darstellt, mit einer Verbindung der Formel (III)

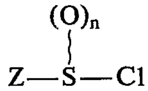

(III)

umsetzt, worin n = 1 oder n = 2, die gewellte Linie ein oder zwei semipolare Doppelbindungen darstellt, die entsprechend n = 1 oder n = 2, das bzw. die Sauerstoffatom(e) mit dem Schwefelatom verbinden, und Z einen Alkylrest mit 1 bis 4 Kohlenstoffatomen, einen monocyclischen Arylrest darstellt, der gegebenenfalls substituiert ist durch entweder eine Alkylgruppe mit 1 bis 4 Kohlenstoffatomen oder durch eine Alkoxygruppe mit 1 bis 4 Kohlenstoffatomen oder durch ein Halogenatom oder durch eine Nitrogruppe

entweder um, wenn n = 1, eine Verbindung mit trans-Struktur im Hinblick auf die Positionen 4 und 5 der allgemeinen Formel (IV)

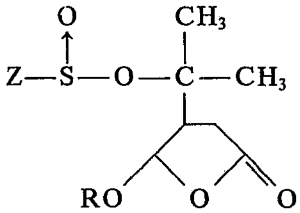

(IV)

zu erhalten, worin R und Z die angegebenen Bedeutungen besitzen, die man mit einem Oxidationsmittel umsetzt, um eine Verbindung der allgemeinen Formel (I)

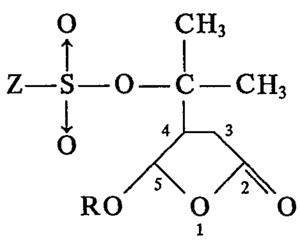

(I)

mit trans-Struktur im Hinblick auf die Positionen 4 und 5, mit der Konfiguration (4SR, 5RS), wenn der Rest des Alkohols R achiral ist oder der Konfiguration (4S, 5R) oder (4R, 5S), wenn der Rest des Alkohols R chiral ist, zu erhalten

oder um, wenn n = 2, eine Verbindung der Formel (I), worin R und Z die vorstehend angegebenen Bedeutungen besitzen, zu erhalten, wobei die Verbindung der allgemeinen Formel (I) die Konfiguration (4SR, 5RS) aufweist, wenn der Rest des Alkohols R achiral ist und die Konfiguration (4S, 5R) oder (4R, 5S) aufweist, wenn der Rest R chiral ist, danach die Verbindung der allgemeinen Formel (I) der Einwirkung eines basischen Mittels unterzieht, wobei die resultierende Cyclisierung unter Retention der Konfiguration, was die Kohlenstoffatome in den Positionen 4 und 5 anbelangt, stattfindet und dazu führt, daß eine Verbindung V mit der Konfiguration (1RS, 4RS, 5SR) einer Verbindung I mit der Konfiguration (4SR, 5RS), eine Verbindung V mit der Konfiguration (1R, 4R, 5S) einer Verbindung I mit der Konfiguration (4S, 5R) und einer Verbindung V mit der Konfiguration (1S, 4S, 5R) einer Verbindung I mit der Konfiguration (4R, 5S) entspricht, danach gewünschtenfalls in saurem Milieu unter Retention der Konfiguration die Ätherfunktion der Verbindung V, worin X einen Alkoholrest darstellt, hydrolysiert.

2. Verfahren gemäß Anspruch 1, dadurch gekennzeichnet, daß man, wenn n = 1, die Kondensation des Sulfinylchlorids III und der Verbindung II in Gegenwart einer tertiären Base und in dem Medium eines Lösungsmittels, ausgewählt unter den halogenierten Kohlenwasserstoffen und aromatischen Kohlenwasserstoffen, durchführt.

3. Verfahren gemäß Anspruch 2, dadurch gekennzeichnet, daß die tertiäre Base Pyridin ist und das Lösungsmittel Methylenchlorid ist.

4. Verfahren gemäß Anspruch 1, dadurch gekennzeichnet, daß das Oxidationsmittel Kaliumpermanganat, Wasserstoffperoxid, Peressigsäure oder p-Nitroperbenzoesäure ist.

5. Verfahren gemäß einem der Ansprüche 1 und 4, dadurch gekennzeichnet, daß die Umsetzung des Oxidationsmittels in dem Medium eines Lösungsmittels oder eines Gemisches von Lösungsmitteln, ausgewählt unter den halogenierten Kohlenwasserstoffen, den aromatischen Kohlenwasserstoffen, der Essigsäure, dem Aceton, den Alkanolen und Wasser, durchgeführt wird.

6. Verfahren gemäß Anspruch 1, dadurch gekennzeichnet, daß man, wenn n = 2, die Kondensation

des Sulfonylchlorids III und der Verbindung II in Gegenwart von Triäthylamin und in dem Medium eines Lösungsmittels, ausgewählt unter den halogenierten Kohlenwasserstoffen' und den aromatischen Kohlenwasserstoffen, durchführt.

7. Verfahren gemäß Anspruch 6, dadurch gekennzeichnet, daß das Lösungsmittel Benzol ist.

8. Verfahren gemäß Anspruch 1, dadurch gekennzeichnet, daß das basische Mittel, dessen Einwirkung man die Verbindung I unterzieht, ein Alkalihydroxid ist.

9. Verfahren gemäß Anspruch 1 oder 8, dadurch gekennzeichnet, daß das basische Mittel nach der als Phasentransferkatalyse bezeichneten Methode verwendet wird.

10. Verfahren gemäß Anspruch 9, dadurch gekennzeichnet, daß bei der als Phasentransferkatalyse bezeichneten Methode das Lösungsmittel Methylenchlorid ist, der Katalysator Triäthylbenzylammoniumchlorid ist, das basische Mittel Natronlauge ist, und daß man in Gegenwart von Wasser arbeitet.

**Claims for the contracting States: BE, CH, DE, GB, IT, LI, NL, SE**

1. In trans form at positions 4 and 5, of (4SR, 5RS), (4S, 5R) or (4R, 5S) configuration, the compounds with the general formula ($I_A$):

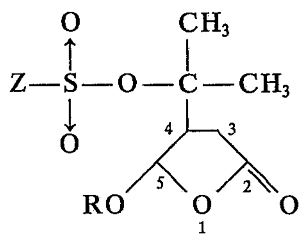

($I_A$)

in which Z represents an alkyl radical with 1—4 carbon atoms, a monocyclic aryl radical possibly substituted either by an alkyl group with 1—4 carbon atoms, or by an alkoxyl group with 1—4 carbon atoms, or by a halogen atom, or by a nitro group and R represents the organic residue of menthol or of meta-phenoxybenzyl alcohol.

2. Preparation process for the cyclopropane derivatives of (1RS, 4RS, 5SR), (1S, 4S, 5R) or (1R, 4R, 5S) configuration with the general formula (V):

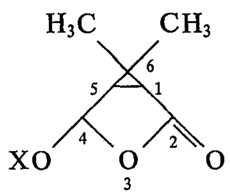

(V)

in which X represents a hydrogen atom or the residue R of an R—OH alcohol possibly chiral, characterized in that a compound with the general formula (II):

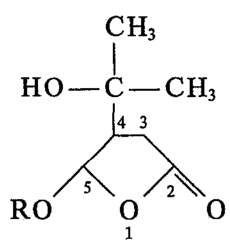

(II)

in which R represents the organic residue of an alcohol R—OH, possibly chiral, the compound (II) being of trans structure at positions 4 and 5 and in the case where R is the residue of an achiral alcohol having a (4 SR, 5 RS) configuration and in the case where R is the residue of a chiral alcohol having a (4 S, 5 R) or (4 R, 5 S) configuration, is made to react with a compound with the formula (III)

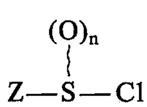

(III)

in which n = 1 or n = 2, the wavy line represents one or two semi-polar bonds linking the oxygen atom(s) to the sulphur atom, according to whether n = 1 or n = 2, and Z represents an alkyl radical with 1—4 carbon atoms, a monocyclic aryl radical possibly substituted either by an alkyl group with 1—4 carbon

atoms, or by an alkoxy group with 1–4 carbon atoms, or by a halogen atom, or by a nitro group,
– either so as to obtain when n=1, a compound with trans structure at positions 4 and 5, with the general formula (IV)

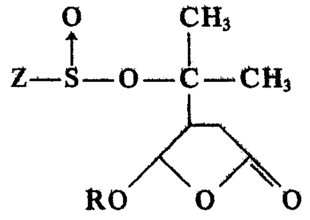

(IV)

in which R and Z retain their previous significance, which is made to react with an oxidizing agent, so as to obtain a compound with the general formula (I):

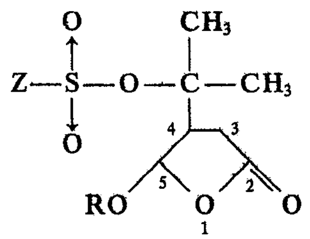

(I)

with trans structure at positions 4 and 5, of (4SR, 5RS) configuration if the R alcohol residue is achiral or of (4S, 5R) or (4R, 5S) configuration if the alcohol residue R is chiral,
– or so as to obtain when n=2, a compound with the general formula (I) in which R and Z retain their previous significance, the compound with the general formula (I) being of (4SR, 5RS) configuration if the alcohol residue R is achiral and of (4S, 5R) or (4R, 5S) configuration if the residue R is chiral, then a compound with the general formula (I) is submitted to the action of a basic agent, the resultant cyclization taking place with configuration retention as regards the carbons at positions 4 and 5 and making a compound (V) of (1RS, 4RS, 5SR) configuration correspond to a compound (I) of (4SR, 5RS) configuration, a compound (V) of (1R, 4R, 5S) configuration correspond to a compound (I) of (4S, 5R) configuration and a compound (V) of (1S, 4S, 5R) configuration correspond to a compound (I) of (4R, 5S) configuration then, if desired the ether function of compound (V) in which X is an alcohol residue is hydrolyzed in an acid medium, with configuration retention.

3. Process according to Claim 2, characterized in that when n=1, the condensation of the sulphinyl chloride III and of the compound (II) is carried out in the presence of a tertiary base and in a solvent chosen from the group constituted by halogenated hydrocarbons and aromatic hydrocarbons.

4. Process according to Claim 3, characterized in that the tertiary base is pyridine and the solvent is methylene chloride.

5. Process according to Claim 2, characterized in that the oxidizing agent is potassium permanganate, hydrogen peroxide, peracetic acid or paranitroperbenzoic acid.

6. Process according to one of Claims 2 and 5, characterized in that the action of the oxidizing agent is carried out in a solvent or a mixture of solvents chosen from the group constituted by halogenated hydrocarbons, aromatic hydrocarbons, acetic acid, acetone, alkanols, and water.

7. Process according to Claim 2, characterized in that when n=2, the condensation of the sulphonyl chloride III and of the compound (II) is carried out in the presence of triethylamine and in a solvent chosen from the group constituted by halogenated hydrocarbons and aromatic hydrocarbons.

8. Process according to Claim 7, characterized in that the solvent is benzene.

9. Process according to Claim 2, characterized in that the basic agent to which the compound with the formula (I) is submitted is an alkaline hydroxide.

10. Process according to Claim 2 or 9, characterized in that the basic agent is utilized according to the method called catalysis by phase transfer.

11. Process according to Claim 10, characterized in that, in the said method of catalysis by phase transfer, the solvent is methylene chloride, the catalyst is triethylbenzylammonium chloride, the basic agent is sodium hydroxyde and the operation is carried out in the presence of water.

**Claims for the contracting State: AT**

1. Preparation process for the cyclopropane derivatives of (1RS, 4RS, 5SR), (1S, 4S, 5R) or (1R, 4R, 5S) configuration with the general formula (V):

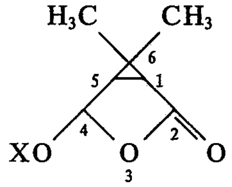

(V)

in which X represents a hydrogen atom or the residue R of an R—OH alcohol possibly chiral, characterized in that a compound with the general formula (II):

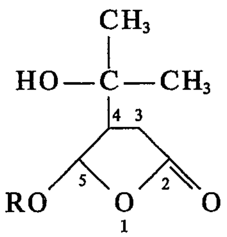

(II)

in which R represents the organic residue of an R—OH alcohol possibly chiral, the compound (II) being of trans structure at positions 4 and 5, and having in the case where R is the residue of an achiral alcohol a (4 SR, 5 RS) configuration, and in the case where R is the residue of a chiral alcohol a (4 S, 5 R) or (4 R, 5 S) configuration, is made to react with a compound with the formula (III):

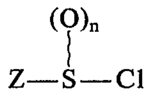

(III)

in which n = 1 or n = 2, the wavy line represents one or two semi-polar bonds linking the oxygen atom(s) to the sulphur, according to whether n = 1 or n = 2, and Z represents an alkyl radical with 1 – 4 carbon atoms, a monocyclic aryl radical possibly substituted either by an alkyl group with 1 – 4 carbon atoms, or by an alkoxyl group with 1 – 4 carbon atoms, or by a halogen atom, or by a nitro group,

– either so as to obtain when n = 1, a compound of trans structure at positions 4 and 5, with the general formula (IV):

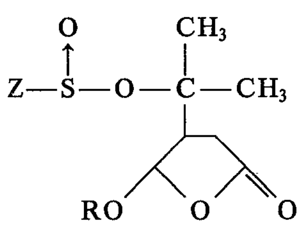

(IV)

in which R and Z retain their previous significance which is made to react with an oxidizing agent, so as to obtain a compound with the general formula (I):

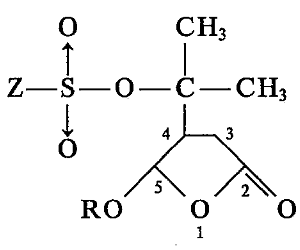

(I)

with trans structure in positions 4 and 5, of (4SR, 5RS) configuration if the alcohol residue R is achiral or of (4S, 5R) or (4R, 5S) configuration if the alcohol residue R is chiral,

– or so as to obtain when n = 2, a compound with the general formula (I) in which R and Z retain their previous significance, the compound with the general formula (I) being of (4SR, 5RS) configuration if the alcohol residue R is achiral and of (4S, 5R) or (4R, 5S) configuration if the residue R is chiral, then a compound with the general formula (I) is submitted to the action of a basic agent, the resultant cyclization taking place with configuration retention as regards the corbons in positions 4 and 5 and making a compound (V) of (1RS, 4RS, 5SR) configuration correspond to a compound (I) of (4SR, 5RS) configuration, a compound (V) of (1R, 4R, 5S) configuration correspond to a compound (I) of (4S, 5R)

configuration and a compound (V) of (1S, 4S, 5R) configuration correspond to a compound (I) of (4R, 5S) configuration, then, if desired, the ether function of the compound (V) in which X is an alcohol residue is hydrolyzed in an acid medium with configuration retention.

2. Process according to Claim 1, characterized in that when n = 1, the condensation of the sulphinyl chloride III and of the compound (II) is carried out in the presence of a tertiary base and in a solvent chosen from the group constituted by halogenated hydrocarbons and aromatic hydrocarbons.

3. Process according to Claim 2, characterized in that the tertiary base in pyridine and the solvent is methylene chloride.

4. Process according to Claim 1, characterized in that the oxidizing agent is potassium permanganate, hydrogen peroxide, peracetic acid of paranitroperbenzoic acid.

5. Process according to one of the Claims 1 and 4, characterized in that the action of the oxidizing agent is carried out in a solvent or mixture of solvents chosen from the group constituted by halogenated hydrocarbons, aromatic hydrocarbons, acetic acid, acetone, alkanols, and water.

6. Process according to Claim 1, characterized in that, when n = 2, the condensation of the sulphonyl chloride III and of the compound (II) is carried out in the presence of triethylamine and in a solvent chosen from the group constituted by halogenated hydrocarbons and aromatic hydrocarbons.

7. Process according to Claim 6, characterized in that the solvent is benzene.

8. Process according to Claim 1, characterized in that the basic agent to which the compound with the fomula (I) is submitted is an alkaline hydroxide.

9. Process according to Claim 1 or 8, characterized in that the basic agent is utilized according to the method called catalysis by phase transfer.

10. Process according to Claim 9, characterized in that in the said method of catalysis by phase transfer, the solvent is methylene chloride, the catalyst is triethylbenzylammonium chloride, the basic agent is sodium hydroxyde and the operation is carried out in the presence of water.